(19) Europäisches Patentamt
European Patent Office
Office européen des brevets

(11) **EP 4 371 480 A1**

(12) **EUROPEAN PATENT APPLICATION**

(43) Date of publication:
**22.05.2024 Bulletin 2024/21**

(51) International Patent Classification (IPC):
**A61B 5/12** *(2006.01)*

(21) Application number: **23211048.6**

(52) Cooperative Patent Classification (CPC):
**A61B 5/123**

(22) Date of filing: **21.11.2023**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.11.2022 EP 22208537**

(71) Applicant: **Interacoustics A/S
5500 Middelfart (DK)**

(72) Inventors:
• **SANCHEZ-LOPEZ, Raul
DK-5500 Middelfart (DK)**
• **LAUGESEN, Søren
DK-5500 Middelfart (DK)**
• **ZAAR, Johannes
DK-2765 Smørum (DK)**
• **SIMONSEN, Lisbeth Birkelund
DK-5500 Middelfart (DK)**

(74) Representative: **Demant
Demant A/S
Kongebakken 9
2765 Smørum (DK)**

(54) **SYSTEM FOR ESTIMATING A HEARING ABILITY OF A TEST SUBJECT**

(57)     System for estimating a hearing ability of a test subject is provided. The system comprises at least one output unit comprising a transducer, the acoustic output unit being configured to provide at least one stimulus into an ear of the test subject via said transducer, a test subject interface for detecting a test subject input and for providing an output to said test subject, a stimulus generator configured to generate said at least one stimulus, where the stimulus generator is configured to provide a plurality of consecutive trials according to a test protocol, where the test protocol is created to estimate a hearing ability of test subject, and where each trial comprises two reference stimuli and one test stimulus, the test stimulus being either a target stimulus or a reference stimulus in accordance with said test protocol, and where the test subject input comprising two alternative options, one corresponding to the target stimulus and the other to the reference stimulus, and where the stimulus generator is configured to provide a trial in response to a detected test subject input, and an analysis unit for analysing a correspondence between each of the provided trials and the following test subject input, and where the analysis unit is configured to adjust said test protocol according to said analysed correspondence. Further, a method of estimating a hearing ability of a test subject is provided.

FIG. 1

EP 4 371 480 A1

**Description**

<u>SUMMARY</u>

The present application relates to a system for estimating a hearing ability of a test subject.

The present application further relates to a method of estimating a hearing ability of a test subject.

The present application further relates to a data processing system comprising a processor and program code means for causing the processor to perform at least some of the steps of the method.

<u>A System</u>

A version of the STM (Spectro-Temporal Modulation) test is called the Audible Contrast Threshold (ACT) test. In the ACT test, an audiologist controls a contrast level (the amount of modulation) of a target signal and controls at which times the modulation is presented to a test subject (e.g., a patient) within a sequence of reference noise.

To determine the threshold, the audiologist starts at the highest contrast level and approximates the threshold using the so-called Hughson-Westlake procedure [1]. If the target stimulus is detected, the contrast level is decreased by two steps (descending approximation). If the target stimulus is not detected, the contrast level is increased by one step (ascending approximation). The procedure stops when three out of the last five detections in the ascending approximation correspond to the same contrast level. The detections in the ascending approximation are the so-called threshold candidates, and the presentations, with or without response, that contain the last five threshold candidates is the threshold candidate window (TCW). The final ACT corresponds to a specific point of the psychometric function, fitted to the trials contained in the TCW.

It is indicated that this test can accurately predict the speech reception performance of individual hearing-impaired (HI) listeners equipped with hearing aids in a speech test [2].

However, by having an audiologist conduct the test, the audiologist necessarily has to focus his/her attention on the equipment and less on the test subject.

Therefore, there is a need for optimizing the test so that the audiologist is relieved at least partly from operating the test/equipment.

The current application is concerned with the implementation of a user-operated ACT (UACT) test that does not require a person, e.g. an audiologist/hearing care professional (HCP), to conduct the test, thus resulting in more time to serve the test subject.

In an aspect of the present application, a system for estimating a hearing ability of a test subject is provided.

For example, the system may be suitable for assessing supra-threshold (audible) hearing abilities of a test subject.

The system comprises at least one output unit comprising a transducer.

The (acoustic) output unit being configured to provide at least one stimulus into an ear of the test subject via said transducer.

For example, the transducer may comprise a receiver (loudspeaker) for providing the stimulus as an acoustic signal to the test subject.

The system comprises a test subject interface for detecting a test subject input (i.e., a response from the test subject) and for providing an output to said test subject.

The system comprises a stimulus generator configured to generate said at least one stimulus.

The stimulus generator is configured to provide a plurality of consecutive trials according to a test protocol, where the test protocol is created to estimate a hearing ability of test subject, and where each trial comprises two reference stimuli and one test stimulus, the test stimulus being either a target stimulus or a reference stimulus in accordance with said test protocol.

The test subject input comprising two alternative options, one corresponding to the target stimulus and the other to the reference stimulus.

The term, corresponding to the target stimulus and the other to the reference stimulus, may refer to that an option for estimating/indicating that the stimulus is the target stimulus and an option for estimating/indicating that the stimulus is the reference stimulus, is provided.

For example, the options (e.g., areas, such as buttons, on a screen) may correspond to target and reference (i.e., state 'target' and 'reference'), or may state 'Yes' and 'No', etc.

For example, the test subject has to indicate whether the test stimulus (e.g., the second stimulus) is a reference stimulus or a target stimulus.

The stimulus generator may be configured to provide a next trial in response to a detected test subject input.

The system comprises an analysis unit for analysing a correspondence between each of the provided trials and the following test subject input, and where the analysis unit is configured to adjust said test protocol according to said analysed correspondence.

Thereby, a UACT test that minimizes or at best eliminates the need for an audiologist/HCP, to conduct the test is provided, thus resulting in more time to serve the test subject.

For example, since the audiologist is not part of the process of obtaining the UACT, the test protocol should preferably take the following points into account:

1. The tasks for the test subject should be easy.
2. The test protocol should adapt the difficulty to the test subject's performance, and alternate stimuli above and below the threshold.
3. The test protocol should include "catch trials" as control for the test subject's performance.
4. The test protocol should include "human intelligence" based on observed behaviour of audiologists when using the manual ACT.

For example, the procedure should provide a reliable and valid threshold estimate in no more than 25 trials (excluding possible trails where the test stimulus is a reference stimulus, i.e., a catch trial).

The test protocol may be divided into at least two phases.

The at least two phases may comprise a pre-test protocol and an actual test protocol.

For example, the pre-test protocol may be conducted prior to initiation of the actual test protocol to obtain a (preliminary) estimation of said hearing ability of the test subject.

For example, the actual test protocol may be conducted to estimating a (an actual) hearing ability of a test subject based on said (preliminary) estimation. Said estimation from the pre-test protocol may be updated throughout the actual test protocol.

The target stimulus may comprise a spectro-temporally modulated audio signal.

The modulation depth of said spectro-temporally modulated audio signal may define the contrast level (CL) of the target stimulus.

The target stimulus may comprise a combined test audio signal and noise audio signal.

The sound pressure level between the test audio signal and the noise audio signal may define a signal-to-noise ratio (SNR) of the target stimulus.

The reference stimulus may define an unmodulated audio signal.

The reference stimulus may comprise a noise audio signal.

The term, the analysis unit being configured to adjust said test protocol according to said analysed correspondence, may comprise adaptively updating said estimated hearing ability.

The stimulus generator may be configured to provide a CL of said target stimulus that is alternating between

- a CL that is higher than said estimated hearing ability of the test subject, and
- a CL that is lower than said estimated hearing ability of the test subject.

The stimulus generator may be configured to provide an SNR of said target stimulus that is alternating between

- an SNR that is higher than said estimated hearing ability of the test subject, and
- an SNR that is lower than said estimated hearing ability of the test subject.

The estimated hearing ability may be adaptively updated in response to said test subject input, where a positive test subject input is identical to an identification of said target stimulus or said reference stimulus.

The estimated hearing ability may be adaptively updated in response to said test subject input, where a negative test subject input is identical to an incorrect identification of said target stimulus or said reference stimulus.

For example, the procedure for obtaining the threshold includes so-called catch trials, where the test stimulus (e.g., the second stimulus) is a reference stimulus. The test subject is expected to indicate that it was not a target stimulus.

For example, if the test subject indicate that he/she heard a target stimulus instead, negative feedback may be shown on the test subject interface indicating that the test subject input was incorrect.

The test subject interface may be configured to provide an output in the form of a test result to said test subject in response to said positive test subject input.

The test subject interface may be configured to provide an output in the form of a test result to said test subject in response to said negative test subject input.

The analysis unit may be configured to determine said test stimulus based on one or more previously determined test subject input.

The test stimulus may be a target stimulus for a number N of said trials and the test stimulus may be a reference stimulus for a number M of said trials.

The number N > number M.

The test protocol may provide a random distribution of said number N and number M of trials. In other words, the test protocol may provide a random distribution of said target stimuli and said reference stimuli.

Each test protocol may be preceded by a pre-test protocol.

At the initiation of each test protocol

- firstly, the stimulus generator may be configured to provide a first trial and a second trial, where the first trial may comprise a test stimulus being a target stimulus and the second trial may comprise a test stimulus being a reference stimulus, and
- secondly, for a fixed number of trials, the stimulus generator may be configured to provide a CL of said target stimulus that is adjusted by:

  - decreasing the CL after a positive test subject input, and
  - increasing the CL after a negative test subject input.

At the initiation of each test protocol

- firstly, the stimulus generator may be configured to provide a first trial and a second trial, where the first trial may comprise a test stimulus being a target stimulus and the second trial may comprise a test stimulus being a reference stimulus, and
- secondly, for a fixed number of trials, the stimulus generator may be configured to provide an SNR of said target stimulus that is adjusted by:

  - decreasing the SNR after a positive test subject input, and
  - increasing the SNR after a negative test subject input.

In response to two or more test subject inputs corresponding to the target stimulus, the analysis unit may be configured to adjust said test protocol to contain that

- the stimulus generator provides that a current (i.e., the next) trial comprises a test stimulus being a reference stimulus, and
- a successive trial comprises a test stimulus being a target stimulus, with a decreased CL, compared to the most previous trial comprising a target stimulus.

For example, the successive trial may comprise a test stimulus being a target stimulus with a CL decreased by 6 dB nCL below the current threshold estimate.

In response to two or more test subject inputs corresponding to the target stimulus, the analysis unit may be configured to adjust said test protocol to contain that

- the stimulus generator provides that a current (i.e., the next) trial comprises a test stimulus being a reference stimulus, and
- a successive trial comprises a test stimulus being a target stimulus, with a decreased SNR, compared to the most previous trial comprising a target stimulus.

For example, the successive trial may comprise a test stimulus being a target stimulus with an SNR decreased by 6 dB below the current threshold estimate.

For example, this may be seen as the patient responds "yes/target" various consecutive times, that suggests that it is either too easy for them or they are just lying. Then, first a reference stimulus to check if they are putting their attention on the test is provided, followed by a target stimulus with decreased CL/SNR so, if we are far from threshold, the procedure can be corrected.

For example, two or more test subject inputs may refer to 2, 4, 6, or another number of inputs. Preferably, it may be in response to 4 test subject inputs.

A test subject input corresponding to the target stimulus, may refer to that the test subject indicates that the test stimulus is a target stimulus.

A test subject input corresponding to the reference stimulus, may refer to that the test subject indicates that the test stimulus is a reference stimulus.

In response to two or more successive identical test subject inputs corresponding to the reference stimulus, the analysis unit may be configured to adjust said test protocol to contain that

- the stimulus generator provides that a current trial comprises a target stimulus with an increased CL compared to the most previous trial comprising a target stimulus.

For example, the current trial may comprise a target stimulus with a CL increased with 6 dB nCL above the current threshold estimate. Thereby, the test subject is helped to remember how the target stimulus sounds like.

In response to two or more successive identical test subject inputs corresponding to the reference stimulus, the analysis unit may be configured to adjust said test protocol to contain that

- the stimulus generator provides that a current trial comprises a target stimulus with an increased SNR compared to the most previous trial comprising a target stimulus.

For example, the current trial may comprise a target stimulus with an SNR increased with 6 dB above the current threshold estimate. Thereby, the test subject is helped to remember how the target stimulus sounds.

For example, two or more successive identical test subject inputs may refer to 2, 4, 6, or another number of inputs. Preferably, it may be in response to 4 successive identical test subject inputs.

The analysis unit may be configured to estimate an audible contrast threshold or a hearing-in-noise threshold of the test subject from a psychometric function derived from the CL of said plurality of target stimuli.

The analysis unit may be configured to estimate an audible contrast threshold or a hearing-in-noise threshold of the test subject from a psychometric function derived from the SNR of said plurality of target stimuli.

The analysis unit may comprise a neural network. The analysis unit may be configured to estimate an audible contrast threshold or a hearing-in-noise threshold of the test subject by use of said neural network.

The analysis unit may be based of Bayes theorem. The procedure may be configured to select the CL or SNR of the next target stimuli to be the level with more information gain [3].

Use

In an aspect, use of a system as described above, and in the claims, is moreover provided.

A method

In an aspect, a method of estimating a hearing ability of a test subject is furthermore provided by the present application.

The method comprises providing a plurality of consecutive trials generated by a stimulus generator, according to a test protocol, into an ear of a test subject via a transducer of at least one output unit.

The test protocol may be based on an estimated hearing ability of the test subject.

Each trial comprises two reference stimuli and one test stimulus.

Accordingly, each trial may comprise a triplet of the three stimuli.

For example, the first and the third stimuli may always be a reference stimulus (e.g., unmodulated noise signal), while the second stimulus may be either a reference stimulus or a target stimulus (e.g., a spectro-temporally modulated noise signal).

The method comprises detecting a test subject input via a test subject interface.

The method may comprise providing a trial by the stimulus generator in response to a detected test subject input.

The test stimulus being either a target stimulus or a reference stimulus in accordance with said test protocol.

The test subject input comprises two alternative options, one corresponding to the target stimulus and the other to the reference stimulus.

The method comprises analysing a correspondence between each of the provided trials and the following test subject input, by an analysis unit.

The method comprises adjusting said test protocol according to said analysed correspondence, by said analysis unit.

The method may further comprise (preliminary) estimating said hearing ability of the test subject in a pre-test protocol carried out before initiation of said test protocol.

The (preliminary) estimation may be updated throughout the test protocol.

It is intended that some or all of the structural features of the system described above, or in the claims can be combined with embodiments of the method, when appropriately substituted by a corresponding process and vice versa. Embodiments of the method have the same advantages as the corresponding system.

A computer readable medium or data carrier

In an aspect, a tangible computer-readable medium (a data carrier) storing a computer program comprising program code means (instructions) for causing a data processing system (a computer) to perform (carry out) at least some (such as a majority or all) of the (steps of the) method described above, and in the claims, when said computer program is executed on the data processing system is furthermore provided by the present application.

A computer program

A computer program (product) comprising instructions which, when the program is executed by a computer, cause the computer to carry out (steps of) the method described above, and in the claims is furthermore provided by the present application.

A data processing system

In an aspect, a data processing system comprising a processor and program code means for causing the processor to perform at least some (such as a majority or all) of the steps of the method described above, and in the claims is furthermore provided by the present application.

An auxiliary device

In a further aspect, a system as described above, and in the claims, AND comprising an auxiliary device is moreover provided.

The system may be adapted to establish a communication link between an operator device of the system (e.g., comprising the analysis unit and/or the signal generator) and the auxiliary device (e.g., comprising the test subject interface) to provide that information (e.g., control and status signals) can be exchanged or forwarded from one to the other.

The system may be adapted to establish a communication link between the operator device and an audio device (e.g., comprising the output unit, e.g., a headset and/or earphones) to provide that information (e.g., the stimuli) can be exchanged or forwarded from one to the other.

The auxiliary device may comprise a remote control, a smartphone, or other portable electronic device, such as a tablet or the like.

The communication link may be a wired or wireless link, e.g., based on Bluetooth or some other standardized scheme.

An APP

In a further aspect, a non-transitory application, termed an APP, is furthermore provided by the present disclosure. The APP comprises executable instructions configured to be executed, e.g., on the auxiliary device, to implement the test subject interface described above, and in the claims. The APP may be configured to run on a remote control, a smartphone, or other portable electronic device, such as a tablet or the like, allowing communication with said operator device.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 shows a system for testing hearing ability of a test subject.

FIG. 2 shows a flow diagram for the method of testing hearing ability of a test subject.

FIG. 3 shows a flow diagram of a modified Hughson-Westlake procedure.

FIG. 4A and 4B show the relationship between the psychometric function ($\Psi$) and the information gain function (IG).

FIG. 5 shows the development of a test protocol comprising 25 trials.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the system and method are described by various blocks, functional units, modules, components, steps, processes, algorithms, etc. (collectively referred to as "elements"). Depending upon particular application, design constraints or other reasons, these elements may be implemented using electronic hardware, computer program, or any combination thereof.

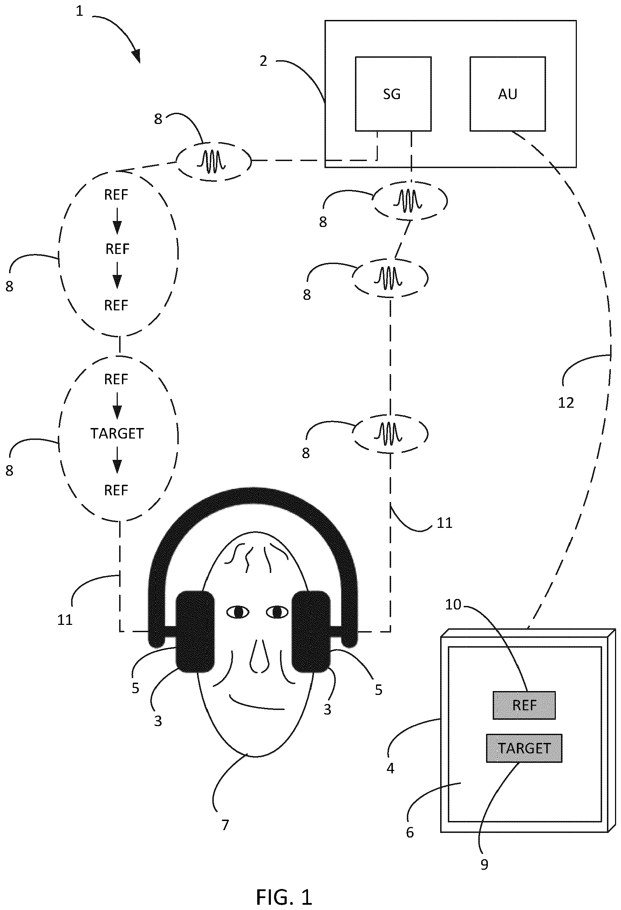

FIG. 1 shows a system for testing hearing ability of a test subject.

In FIG. 1, it is shown that the system 1 may be divided up into more than one device. However, it is foreseen that the system 1 may be just one device. Thus, it is foreseen that one or both of the stimulus generator SG and the analysis unit AU may be incorporated into an audio device, such as a headset.

Accordingly, the system 1 is shown to comprise an operator device 2, an audio device 3, and an auxiliary device 4.

The operator device 2 may comprise the stimulus generator SG and the analysis unit AU. The audio device 3 may comprise at least one output unit 5. In FIG. 1, two output units 5 are shown.

The auxiliary device 4 may comprise a test subject interface 6.

The stimulus generator SG may be configured to generate at least one stimulus and to transmit said at least one stimulus to the audio device 3.

The stimulus generator SG is shown to be configured to provide a plurality of consecutive trials 8 according to a test protocol. In FIG. 1, three consecutive trials 8 are shown to be presented to each of the two output units 5 of the audio device 3. However, the test protocol may include to present several consecutive trials 8, such as 20, 25, 30, etc. trials (excluding catch trails).

The stimulus generator SG may transmit said trials 8 via a wired or wireless connection 11 to the audio device 3.

Each trial 8 may comprise two reference stimuli REF and one test stimulus, the test stimulus being either a target stimulus TARGET or a reference stimulus REF in accordance with said test protocol. In FIG. 1, the first trial 8 presented to the test subject contains a stimulus triplet of reference-target-reference and the subsequent second trial 8 contains a stimulus triplet of reference-reference-reference (a so-called catch trial).

For example, when testing an audible contrast threshold, the target stimulus may be a modulated stimulus (not a combination), and the modulation depth changes according to the test protocol; and it is defined in CL.

For example, alternatively, when testing hearing in noise, the target stimulus is a combination of a test audio signal + noise audio signal, and the level of the test audio signal changes according to the test protocol; and the threshold is defined in SNR.

In FIG. 1, the audio device 3 is illustrated as being a headset that is placed at the ears of a test subject 7, but the audio device 3 may instead be placed at/in only one of the ears, and other earphones or other devices comprising a transducer may alternatively be used. The two output units 5 of the audio device 3 may (each) comprise a transducer, and the acoustic output units 5 may each be configured to provide the at least one stimulus into the ear(s) of the test subject 7 via said transducer. Accordingly, the consecutive trials 8 may be transmitted to the audio device 3 and each of the output units 5 may present the stimuli of the trials 8 into the ears of the test subject 7 via said transducers.

In FIG. 1, the auxiliary device 4 is illustrated as being a tablet, but other (e.g., portable) devices may be used. The test subject interface 6 of the auxiliary device 4 may be configured to detect a test subject input by the test subject 7, and to provide an output to said test subject 7.

It is shown that the test subject input may comprise two alternative options, one corresponding to the target stimulus, e.g. a target-button 9, 'TARGET', the test subject 7 can press in case he/she identifies a target stimulus in the current trial, and another corresponding to the reference stimulus, e.g. a reference-button 10, 'REF', the test subject 7 can press in case he/she identifies a reference stimulus in the current trial.

The stimulus generator SG may be configured to provide/design the next trial 8 in response to a detected test subject input.

The analysis unit AU may be suitable for analysing a correspondence between each of the provided trials 8 and the following test subject input, detected by the auxiliary device 4.

The analysis unit AU may be configured to adjust said test protocol according to said analysed correspondence.

A communication link 12 may exist between the auxiliary device 4 and the analysis unit AU so that information can be transmitted back and forth. For example, the auxiliary device 4 may transmit the test subject input to the analysis unit AU. The analysis unit AU may transmit an output to be presented to the test subject 7 on the test subject interface 6.

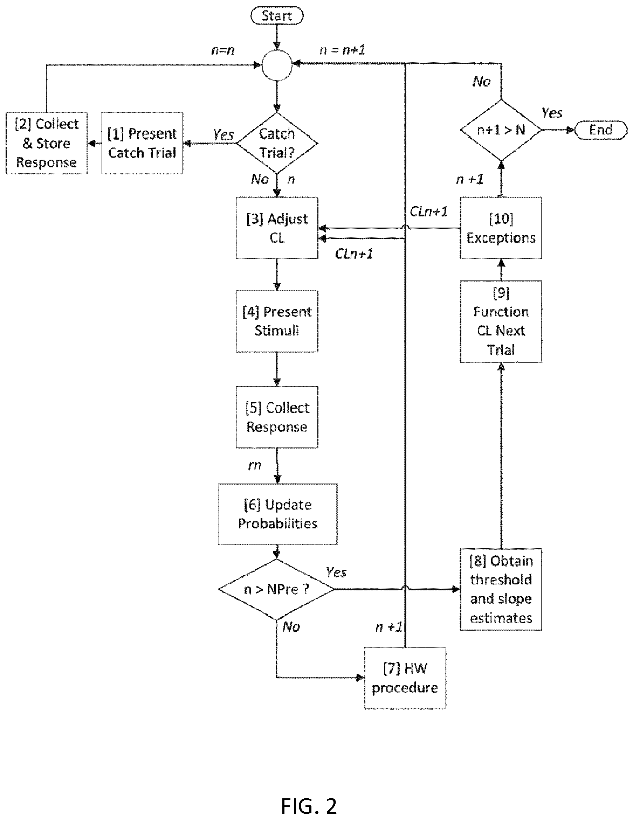

FIG. 2 shows a flow diagram for the method of testing hearing ability of a test subject.

FIG. 2 presents the overall procedure, in other words the test protocol, for testing the hearing ability. Some of the separate steps of the procedure will be described in further detail in the following FIGs.

The test protocol shown in FIG. 2 is divided into two phases, a pre-test protocol and an actual test protocol. For trial numbers n < NPre, a pre-test protocol is run, and for n > NPre, an actual test protocol is run.

At initiation (step 'Start') of the test protocol, the $n^{th}$ trial (stimulus triplet) is either a trial, where the test stimulus is a target stimulus or a reference stimulus (step 'Catch Trial?').

In case the $n^{th}$ trial is a catch trial, the trial is presented in a triplet of reference-reference-reference (step [1]). Secondly, the test subject gives a response (step [2]). The catch trials are randomly assigned at the beginning of the procedure to trials that are between 4 and 8 trials apart. The test subject inputs to the catch trials are stored since it can be an indicator of the reliability of the test protocol.

The catch trial is not counted as one of the n trials of the actual test protocol. Thus, the $n^{th}$ trial is presented again. In case the $n^{th}$ trial is not a catch trial, the CL of the target stimulus is adjusted (step [3]), and the trial is presented to the test subject (step [4]). In the following steps (steps [5] + [6]), the response (i.e., the test subject input) is collected and the probabilities are updated.

For the $n^{th}$ trials where n < NPre, the pre-test protocol is run. The pre-test protocol includes adjusting the CL of the next trial (n+1) (step [3]) based on the modified Hughson-Westlake procedure (step [7]). The modified Hughson-Westlake procedure (step [7]) is described in more detail below. At the end of the pre-test protocol, an estimated hearing ability of the test subject is obtained, that may be used in the actual test protocol.

At n > NPre, the actual test protocol is initiated. During the actual test protocol, the hearing ability estimated in the pre-test protocol is adaptively updated.

For the $n^{th}$ trial, the probability of each possible threshold ($th_a$) and slope ($sl_b$) is updated based on the Bayes rule (step [6]).

Based on the Bayes theorem we may simplify the following equation:

$$p(\lambda_x|CL_n) = p(r_k|\lambda_x)p(\lambda_x|CL_{n-1})$$

where $\lambda_x$ may consist of two parameters: $\lambda_{th}$, which is the estimate of the threshold of a psychometric function, or $\lambda_{sl}$, which is the estimate of the slope of the psychometric function, and $r_k$ are the $K$ responses (1 correct, 0 incorrect), and CL are the contrast levels of a given trial.

To simplify, the probabilities can be understood as:

$$Posterior = likelihood \; x \; Prior$$

So, the posterior probability is the result of the product of the likelihood $p(r_k|\lambda_x)$ and the prior probability (which was the posterior of the previous trial).

In the first trial, the *Prior* probability is uniform, so the probability $P(th_a, sl_b)$ that each term corresponds to a possible value of the threshold $th_a$ and a possible value of the slope $sl_b$ is the same

$$Prior = \begin{bmatrix} P(th_1, sl_1) & \cdots & P(th_A, sl_1) \\ \vdots & \ddots & \vdots \\ P(th_1, sl_B) & \cdots & P(th_A, sl_B) \end{bmatrix}$$

$$th_a = [-4, -3, ..., 16]$$

$$sl_b = [0.5 \; 0.75 \; 1 \; 1.25 \; 1.5]$$

For the $n^{th}$ trial, the CL and the response of the last K trials are used to calculate the likelihood. In this procedure, we make use of $k = [n - 1, n]$, which means, that this is calculated using the current $n$ and previous $n-1$ responses. However, in the first trial $k = [n]$, since only one trial has been presented.

Considering a generic psychometric function:

$$\Psi(th, sl, CL) = \frac{1}{1 + e^{sl(th-CL)}}$$

The likelihood for each possible value of the threshold and slope is calculated as:

$$Likelihood\ (a, b) = \prod_{k}^{K} \Psi(th_a, sl_b, CL_k)^{r_k}\ (1 - \Psi(th_a, sl_b, CL_k))^{(1-r_k)};$$

$$k = [n - 1, \quad n]$$

Therefore, the posterior probability is then computed as the product, term by term, of the Prior and the Likelihood matrices.

$$Posterior = Prior \odot Likelihood$$

Therefore, in the present application, the last two trials (n-1, n) are used for updating the probabilities.

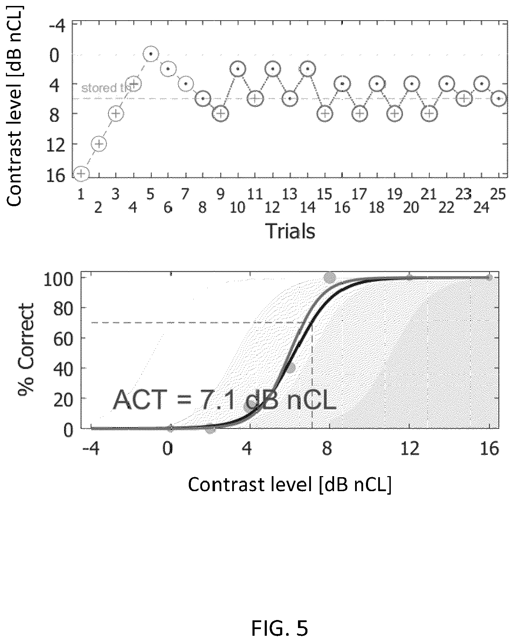

The current estimates of the threshold ($\lambda_{th}$) and of the slope ($\lambda_{sl}$) may then be obtained (step [8], see below) and may be used to calculate the optimal CL for the next trial based on a function of information gain (step [9], see FIG. 5 for further detail). If the previous responses suggest that the test subject's responses are not reliable, an exception of the test protocol may be triggered in order to improve the threshold estimation (step [10]).

The step [8] of obtaining threshold and slope estimates may be achieved by, from the posterior probability, estimating the parameters $\lambda_x$ by simply obtaining the argument of the maximum value of the Posterior matrix (i.e., the row $\alpha$ and the column $\beta$)

$$\alpha, \beta = arg\text{max}\ (Posterior)$$

and then using these indexes with the vector of the possible values of the threshold and slopes

$$\lambda_{th} = th_\alpha;\ \lambda_{sl} = sl_\beta;$$

Based on the experience gained by using the manually administered ACT test, some exceptions to the previously explained test protocol were included as a step [10].

The exceptions are summarized in two actions, which may be taken by the system based on the last X responses, in the form of additional catch trials or presentations above the threshold. For example, the system may use X=4, so $\chi$=[n-3,n].

Additional catch trials: By default, the system may consider 4-5 catch trials assigned randomly to specific trial numbers with 4-8 trials in between. The exception may be two-fold:

a) The first catch trial may always be trial number 2. Thereby, the first trial will present a fully modulated target stimulus and the second trial a reference stimulus (as test stimulus). That is meant to help the test subjects to create their own internal representation.

b) An additional catch trial may be added every time X consecutive test subject inputs in non-catch trials corresponds to a target stimulus (e.g., a 'yes' or 'target' response). If a test subject is systematically pressing the button 'TARGET', that could trigger a catch trial every other trial. A high number of catch trials will be indicative of an unreliable test run.

c) After the additional catch trial, the following trial may then be presented 6 dB nCL below the current threshold estimate $\lambda_{th}$. This is done on the assumption that the test subject might have responded positively (i.e., providing a test subject input corresponding to a target stimulus) to the previous X responses and the actual threshold might be well below the previous presentations. Furthermore, if the test subject is "caught" in the catch trial, his/her response criteria might be more conservative in the following trials. In that case, in the following trial(s) the test subject is expected to provide a test subject input corresponding to a reference stimulus (e.g., by pressing 'REF'). On the other hand, by providing a test subject input corresponding to a target stimulus (e.g., by pressing 'TARGET') might be indicative of a clearly unreliable test subject.

Presentations above the threshold: If a test subject provides an input corresponding to a reference stimulus (e.g., presses 'REF') in X consecutive trials, the following trial may be presented 6 dB nCL above the current threshold estimate $\lambda_{th}$. This is done on the assumption that the test subject has "lost" his/her internal reference and needs a reminder of "how the target stimulus sounds like". After that trial, the test subject can focus again on identifying target stimuli.

Accordingly, besides the adaptive procedure, exceptions based on the experience reported by audiologists with manual tests have been included in the test protocol. Depending on the test subject's previous responses, the system may trigger additional catch trials or presentations well above threshold to keep the test subject's attention and ensure consistent performance.

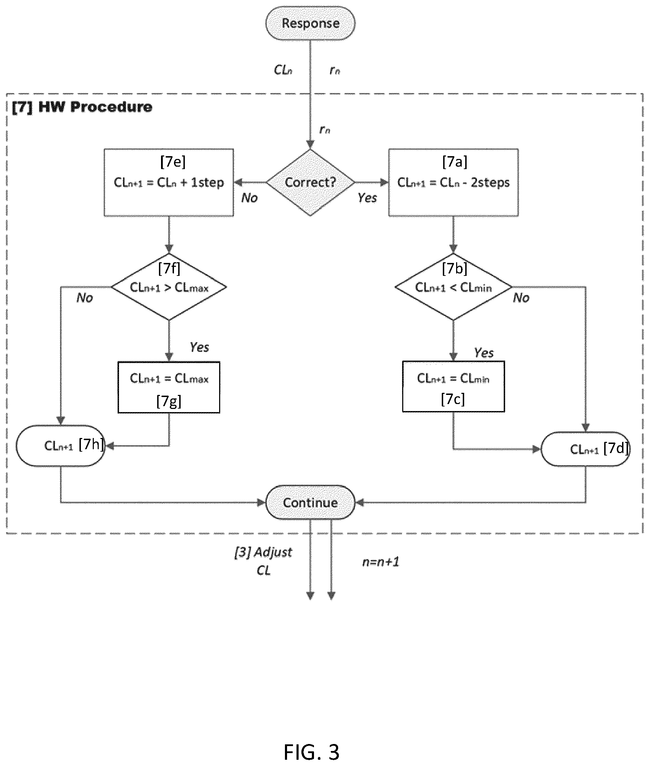

FIG. 3 shows a flow diagram of a modified Hughson-Westlake procedure [1].

If a test subject input (step 'Response') is correct (step 'Correct?'), the CL of the following CL, $CL_{n+1}$, is decreased by two steps, i.e., $CL_{n+1} = CL_n$ - 2steps (step [7a]).

If the $CL_{n+1}$ is below a minimum CL, $CL_{min}$ (step [7b]), the CL is adjusted to said minimum CL (step [7c]), which is output (step 'Continue'). Otherwise, the already determined $CL_{n+1}$ (step [7d]) is output (step 'Continue'), to e.g., adjust the CL (in step [3]).

If the test subject input (step 'Response') is not correct (step 'Correct?'), the CL of the following CL, $CL_{n+1}$, is increased by one step, i.e., $CL_{n+1} = CL_n$ + 1step (step [7e]).

If the $CL_{n+1}$ is above a maximum CL, $CL_{max}$ (step [7f]), the CL is adjusted to said maximum CL (step [7g]), which is output (step 'Continue'). Otherwise, the already determined $CL_{n+1}$ (step [7h]) is output (step 'Continue'), to e.g., adjust the CL (in step [3]).

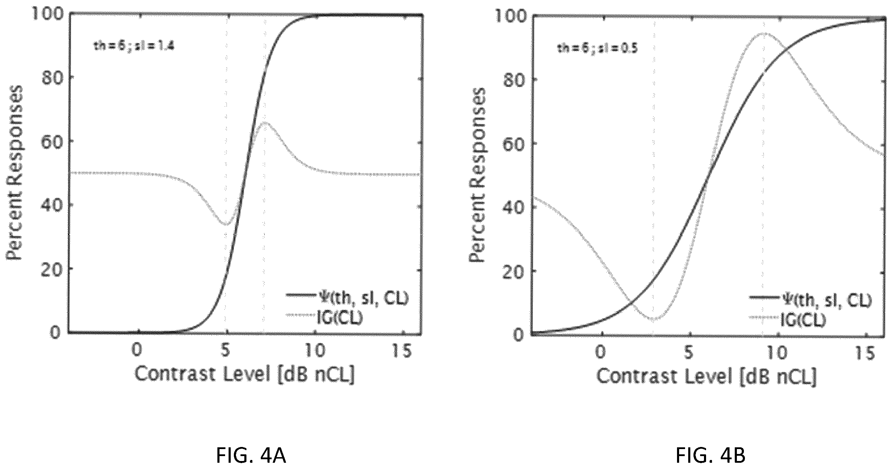

FIG. 4A and 4B show the relationship between the psychometric function ($\Psi$) and the information gain function (IG).

In the present application, a simplified information gain function is used, based on two assumptions:

1) Test subjects going through a test protocol (e.g., performing a yes/no task) would keep more consistently their attention if the stimuli are presented alternatingly a bit above and a bit below the (presently estimated) threshold.
2) The slope of the psychometric function controls the difference in contrast level between these two points (i.e., the stimuli a bit above and a bit below the threshold).

The two points may be estimated at 82% (i.e., a bit above the threshold) and at 18% (i.e., a bit below the threshold) of the psychometric function.

To obtain an estimate of the 82% and 18% points of the psychometric function, the derivative of the psychometric function first has to be calculated with respect of the slope:

$$\frac{\partial \Psi(th, sl, CL)}{\partial sl} = -e^{sl(th-CL)}\frac{th - CL}{(1 + e^{sl(th-CL)})^2}$$

The information gain (IG) function is then the (expression) defined for $th = \lambda_{th}$ and $sl = \lambda_{sl}$

$$IG\,(CL) = \frac{\partial \Psi(th, sl, CL)}{\partial sl}\Bigg|_{th=\lambda_{th}\,;\,sl=\lambda_{sl}} \quad for\ CL = th_a$$

The corresponding IG function decreases with a minimum at the 18% of the psychometric function and then increases with a maximum at the 82%.

In FIGS. 4A and 4A, it is exemplified how the psychometric function ('$\Psi$' solid line) and the information gain function ('IG' dotted line) are related.

FIG. 4A depicts a test run with very consistent responses from the test subject so the maximum and minimum values are close to each other and the distance between $CL_n$ and $CL_{n+1}$ (min and max of IG) would be small (~2dB nCL). The threshold is determined to be 6 dB and the slope of the psychometric function is 1.4.

FIG. 4B depicts a less consistent test run where the $CL_n$ and $CL_{n+1}$ are further apart (~4 dB nCL). The threshold is determined to be 6 dB and the slope of the psychometric function is 0.5.

To use an alternating presentation of said two points (i.e., 82% and 18%), the minimum may be presented in the even trials and the maximum in the odd trials.

$$\begin{cases} if\ n\ \text{is} \begin{cases} even, & CL_{n+1} = \text{minarg}(IG(CL)) \\ odd, & CL_{n+1} = \text{maxarg}(IG(CL)) \end{cases} \end{cases}$$

The advantage of this procedure is that the step size between $CL_n$ and $CL_{n+1}$ depends on the performance reflected in the current estimate of the slope. If in the previous trials the test subject showed consistent responses, the slope will be larger and the step size will be minimum.

If the responses in the previous trials were less consistent, the slope will be smaller and the step size larger. At the end of all N trials, the audible contrast threshold will result from the psychometric function fitted to the data. Since the procedure collects the responses of many trials at the estimated 18% and 82% points, the resulting threshold is more robust and efficient than another adaptive procedure where many presentations can be well below or above the 18% or 82% points. Importantly, the $CL_{n+1}$ can only be adjusted to a value according to the step size, which in this case is 2 dB nCL.

FIG. 5 shows the development of a test protocol comprising 25 trials.

In the top graph of FIG. 5, the nCL of the target stimuli for each of the 25 trials are shown. A dotted line at 6 dB indicates a stored threshold, which may e.g., have been determined during a pre-test protocol prior to the test protocol shown in FIG. 5.

For the first four stimuli, the test subject provides the input that a target stimulus is identified (indicated by the '+'). For each step, the nCL is decreased by 4 dB (2 step sizes). At the fifth stimulus, the test subject provides the input that the target stimulus is not identified (indicated by the '.'), which is also the case for the following three stimuli. For each of these steps, the CL is increased by 2 dB. The test subject identifies the target stimulus again at an nCL of 8 dB. Afterwards, the target stimuli fluctuate between an nCL below the threshold and an nCL above the threshold.

In the bottom graph of FIG. 5, the percent of correct responses as function of nCL is shown in the form of a psychometric function fitted to the data of the test. The resulting audible contrast threshold may be determined from the psychometric function at the end of the test protocol. The threshold is estimated to be 7.1 dB as indicated by the dotted lines.

It is intended that the structural features of the devices and system described above, either in the detailed description and/or in the claims, may be combined with steps of the method, when appropriately substituted by a corresponding process.

As used, the singular forms "a," "an," and "the" are intended to include the plural forms as well (i.e., to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

REFERENCES

[1] Carhart, R., & Jerger, J. F. (1959). Preferred Method For Clinical Determination Of Pure-Tone Thresholds. Journal of Speech and Hearing Disorders, 24(4), 330-345.

[2] Zaar, J., Simonsen, L. B., Behrens, T., Dau, T., & Laugesen, S. (2019). Investigating the relationship between spectro-temporal modulation detection, aided speech perception, and noise reduction preference. ISAAR.

[3] Remus, J. J., & Collins, L. M. (2008). Comparison of adaptive psychometric procedures motivated by the Theory of Optimal Experiments: Simulated and experimental results. The Journal of the Acoustical Society of America, 123(1), 315-326. https://doi.org/10.1121/1.2816567

**Claims**

1. System for estimating a hearing ability of a test subject, where the system comprises

    - at least one output unit comprising a transducer, the acoustic output unit being configured to provide at least one stimulus into an ear of the test subject via said transducer,
    - a test subject interface for detecting a test subject input and for providing an output to said test subject,
    - a stimulus generator configured to generate said at least one stimulus,
    - where the stimulus generator is configured to provide a plurality of consecutive trials according to a test protocol, where the test protocol is created to estimate a hearing ability of test subject, and where each trial comprises two reference stimuli and one test stimulus, the test stimulus being either a target stimulus or a reference stimulus in accordance with said test protocol, and
    - where the test subject input comprising two alternative options, one corresponding to the target stimulus and the other to the reference stimulus, and where the stimulus generator is configured to provide a trial in response to a detected test subject input, and
    - an analysis unit for analysing a correspondence between each of the provided trials and the following test subject input, and where the analysis unit is configured to adjust said test protocol according to said analysed correspondence.

2. System according to claim 1, wherein the test protocol is divided into two phases, a pre-test protocol and an actual test protocol.

3. System according to any one of claims 1-2, wherein the target stimulus comprises a spectro-temporally modulated audio signal, the modulation depth defines the contrast level (CL) of the target stimulus.

4. System according to any one of claims 1-2, wherein the target stimulus comprises a combined test audio signal and noise audio signal, where the sound pressure level between the test audio signal and the noise audio signal defines a signal-to-noise ratio (SNR) of the target stimulus.

5. System according to any one of the preceding claims, wherein the reference stimulus comprises a noise audio signal.

6. System according to any one of the preceding claims, wherein, the analysis unit being configured to adjust said test protocol according to said analysed correspondence, comprises adaptively updating said estimated hearing ability.

7. System according to any one of the preceding claims, wherein the stimulus generator is configured to provide a CL or SNR of said target stimulus that is alternating between

    - a CL or SNR, respectively, that is higher than said estimated hearing ability of the test subject, and
    - a CL or SNR, respectively, that is lower than said estimated hearing ability of the test subject.

8. System according to any one of claims 6-7, wherein the estimated hearing ability is adaptively updated in response to said test subject input, where

    - a positive test subject input is identical to an identification of said target stimulus or said reference stimulus, and
    - a negative test subject input is identical to an incorrect identification of said target stimulus or said reference stimulus.

9. System according to claim 8, wherein the test subject interface is configured to provide an output in the form of a test result to said test subject in response to said positive or negative test subject input.

10. System according to any one of the preceding claims, wherein the analysis unit is configured to determine said test stimulus based on one or more previously determined test subject input.

11. System according to any one of the preceding claims, wherein the test stimulus being a target stimulus for a number N of said trials and the test stimulus being a reference stimulus for a number M of said trials, where N > M.

12. System according to claim 11, wherein the test protocol provides a random distribution of said number N and M of trials.

13. System according to any one of claims 8-12, wherein, at the initiation of each test protocol,

- firstly, the stimulus generator is configured to provide a first trial and a second trial, where the first trial comprises a test stimulus being a target stimulus and the second trial comprises a test stimulus being a reference stimulus, and
- secondly, for a fixed number of trials, the stimulus generator is configured to provide a CL or SNR of said target stimulus that is adjusted by:

- decreasing the CL or SNR after a positive test subject input, and
- increasing the CL or SNR after a negative test subject input.

14. System according to any one of claims 8-13, wherein, in response to two or more test subject inputs corresponding to the target stimulus from said test subject, the analysis unit is configured to adjust said test protocol to contain that

- the stimulus generator provides that a current trial comprises a test stimulus being a reference stimulus, and
- a successive trial comprises a test stimulus being a target stimulus with a decreased CL or SNR compared to the most previous trial comprising a target stimulus.

15. System according to any one of claims 3-14, wherein, in response to two or more successive identical test subject inputs corresponding to the reference stimulus from the test subject, the analysis unit is configured to adjust said test protocol to contain that

- the stimulus generator provides that a current trial comprises a target stimulus with an increased CL or SNR, compared to the most previous trial comprising a target stimulus.

16. System according to any one of claims 3-15, wherein the analysis unit is configured to estimate an audible contrast threshold or a hearing-in-noise threshold of the test subject from a psychometric function derived from the CL or SNR of said plurality of the target stimuli.

17. Method of estimating a hearing ability of a test subject, where the method comprises:

- providing a plurality of consecutive trials generated by a stimulus generator according to a test protocol, based on an estimated hearing ability of test subject, into an ear of a test subject via a transducer of at least one output unit,
- where each trial comprises two reference stimuli and one test stimulus,
- detecting a test subject input via a test subject interface,
- providing a trial by the stimulus generator in response to a detected test subject input,
- the test stimulus being either a target stimulus or a reference stimulus in accordance with said test protocol, and where the test subject input comprises two alternative options, one corresponding to the target stimulus and the other to the reference stimulus, and
- analysing a correspondence between each of the provided trials and the following test subject input, by an analysis unit, and
- adjusting said test protocol according to said analysed correspondence, by said analysis unit.

18. Method according to claim 17, wherein the method comprises a preliminary estimation of said hearing ability of the test subject in a pre-test protocol carried out before initiation of said test protocol, and the estimation is updated throughout the test protocol.

19. A data processing system comprising a processor and program code means for causing the processor to perform at least some of the steps of the method of claims 17-18.

FIG. 1

FIG. 2

**Response**

$CL_n$    $r_n$

**[7] HW Procedure**

$r_n$

**Correct?**

**No** → **[7e]**
$CL_{n+1} = CL_n + 1\text{step}$

**Yes** → **[7a]**
$CL_{n+1} = CL_n - 2\text{steps}$

**[7f]**
$CL_{n+1} > CL_{max}$

**No** →

**Yes** ↓
$CL_{n+1} = CL_{max}$
**[7g]**

**[7b]**
$CL_{n+1} < CL_{min}$

**No** →

**Yes** ↓
$CL_{n+1} = CL_{min}$
**[7c]**

$CL_{n+1}$ **[7h]**

$CL_{n+1}$ **[7d]**

**Continue**

*[3] Adjust CL*        *n=n+1*

FIG. 3

FIG. 4B

FIG. 4A

FIG. 5

Europäisches
Patentamt

European
Patent Office

Office européen
des brevets

# EUROPEAN SEARCH REPORT

Application Number

EP 23 21 1048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SANCHEZ-LOPEZ RAUL ET AL.: "IHCON 2022 International Hearing Aid Seminar (IHAS)", INTERNATIONAL HEARING AID RESEARCH CONFERENCE, vol. 2, 14 August 2022 (2022-08-14), XP093018713, * page 19, column 2 - page 20, column 1 * ----- | 1-19 | INV. A61B5/12 |
| X | US 2003/083591 A1 (EDWARDS BRENT W [US] ET AL) 1 May 2003 (2003-05-01) | 1,2,4-19 | |
| A | * paragraphs [0017], [0018], [0029], [0038], [0057], [0058], [0076] - [0078]; figure 4 * ----- | 3 | |
| A | US 2013/303940 A1 (SALY GEORGE L [US]) 14 November 2013 (2013-11-14) * Table 1; paragraphs [0057], [0063], [0064], [0067] * ----- | 1-19 | |

| | | | TECHNICAL FIELDS SEARCHED (IPC) |
|---|---|---|---|
| | | | A61B |

The present search report has been drawn up for all claims

| Place of search | Date of completion of the search | Examiner |
|---|---|---|
| The Hague | 7 February 2024 | Papanikolaou, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 21 1048

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2024

| Patent document cited in search report | | Publication date | Patent family member(s) | | Publication date |
|---|---|---|---|---|---|
| US 2003083591 | A1 | 01-05-2003 | AU | 2002347871 A1 | 22-04-2003 |
| | | | US | 2003083591 A1 | 01-05-2003 |
| | | | WO | 03030619 A2 | 17-04-2003 |
| US 2013303940 | A1 | 14-11-2013 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CARHART, R ; JERGER, J. F.** Preferred Method For Clinical Determination Of Pure-Tone Thresholds. *Journal of Speech and Hearing Disorders,* 1959, vol. 24 (4), 330-345
- **ZAAR, J. ; SIMONSEN, L. B. ; BEHRENS, T ; DAU, T. ; LAUGESEN, S.** Investigating the relationship between spectro-temporal modulation detection, aided speech perception, and noise reduction preference. *ISAAR.,* 2019

- **REMUS, J. J. ; COLLINS, L. M.** Comparison of adaptive psychometric procedures motivated by the Theory of Optimal Experiments: Simulated and experimental results. *The Journal of the Acoustical Society of America,* 2008, vol. 123 (1), 315-326, https://doi.org/10.1121/1.2816567